# EUROPEAN PATENT SPECIFICATION

(11) **EP 3 083 862 B1**
(45) Date of publication and mention of the grant of the patent: **05.08.2020**
(21) Application number: 14822020.5
(22) Date of filing: 16.12.2014
(51) Int. Cl.: A61F 13/02, C09J 133/14, C08K 5/00, C09J 7/38

(54) **ANTIMICROBIAL PRESSURE-SENSITIVE ADHESIVE SYSTEM**
ANTIMIKROBIELLES HAFTKLEBEMITTELSYSTEM
SYSTÈME ADHÉSIF ANTIMICROBIEN SENSIBLE À LA PRESSION

(30) Priority: 20.12.2013 US 201361918907 P
(43) Date of publication of application: 26.10.2016
(73) Proprietor: 3M Innovative Properties Company, St. Paul, MN 55133-3427 (US)
(72) Inventor: LIU, Junkang J., Saint Paul, Minnesota 55133-3427 (US); CONRAD-VLASAK, Deena M., Saint Paul, Minnesota 55133-3427 (US); ALI, Mahfuza B., Saint Paul, Minnesota 55133-3427 (US); STEPANOVA, Narina Y., Saint Paul, Minnesota 55133-3427 (US); JOHNSON, Elizabeth E., Saint Paul, Minnesota 55133-3427 (US)
(74) Representative: Vossius & Partner Patentanwälte Rechtsanwälte mbB
(86) International application number: PCT/US2014/070549
(87) International publication number: WO 2015/095165

(56) References cited:
- EP-A1- 0 965 626
- WO-A1-03/062343
- WO-A2-2011/150001
- US-A1- 2007 149 694

## Description

### FIELD

The present disclosure generally relates to antimicrobial pressure-sensitive adhesive systems, and particularly, to antimicrobial pressure-sensitive adhesive systems for use with medical articles.

### BACKGROUND

Medical adhesives can have many uses, including securing wound dressings, medical devices (e.g., catheters) surgical drapes, tapes, sensors, and the like, as well as skin sealant and liquid sutures, etc.

Pressure-sensitive adhesives have been used for a variety of marking, holding, protecting, sealing, and masking purposes. Pressure-sensitive adhesive can also be useful in the field of skin or medical adhesives, e.g., for use in wound dressings, or the like. The living, sensitive, low surface energy and highly textured surface of skin can present difficulties in adhesion, and the wide variation in the skin surface from individual to individual and from location to location on the same individual can exacerbate these difficulties.

Healthcare associated infections (HAIs) also known as hospital acquired infections have been increasing and have become a public health concern. Patients with HAIs have longer hospital stays contributing to increased health care costs. Use of preventive interventions can reduce inpatient hospital HAIs by 70%. The pressure on hospitals to reduce HAIs is leading to an increased used of hygiene products and an increasing demand for new antimicrobial solutions.

### SUMMARY

Antimicrobials can kill and/or prevent the growth of bacteria. Antimicrobials can be classified into two types: leaching or leachable (e.g., silver (Ag), chlorhexidine gluconate (CHG), and polyhexamethylene biguanide (PHMB)); and non-leaching or non-leachable (e.g., covalently-bound quaternary amines). In some cases, leachable antimicrobials can be associated with concerns of generating adaptive organisms and toxicity by leaching harmful chemicals into ecologic systems. As a result, use of non-leachable antimicrobials can reduce the amount of leachable antimicrobials needed in a formulation to achieve a desired antimicrobial efficacy, while reducing downstream effects of toxicity or bacterial resistance.

The present disclosure generally relates to antimicrobial pressure-sensitive adhesive systems ("PSA systems") comprising an antimicrobial polymer, wherein the antimicrobial moiety is covalently attached to the polymer, such that the antimicrobial moiety is immobilized on the polymer and non-leaching from the polymer. Although the antimicrobial moiety is non-leachable from the polymer it is mobile in the micro-environment to access and kill bacteria. The PSA systems of the present disclosure can further include a secondary (e.g., dispersed) antimicrobial agent for a synergistic effect.

The PSA systems of the present disclosure can be employed in a variety of articles, including, but not limited to, at least one of medical tapes, skin closure tapes, surgical drapes, medical dressings (e.g., intravenous dressings and wound dressings), electrodes, wearable sensors, ostomy pouches, adhesive patches, medical tubing securement devices, first aid bandages.

Use of the antimicrobial PSA systems and resulting antimicrobial articles of the present disclosure can suppress growth of bacteria and prevent the spread of bacteria. These antimicrobial PSA systems and articles of the present disclosure can be used prophylactically, because it is unlikely for pathogens to develop resistance to the quaternary ammonium-based compounds ("quats"). This is at least partially because quats function by binding to bacterial cell membranes and disrupting the barrier function of the membrane, leading to loss of the transmembrane potential, leakage of cytoplasmic contents, and concomitant cell death. The cationic charges in such quats can enhance their affinity to anionic components of the bacterial cell membranes by electrostatic attraction.

"Alkyl" means a linear or branched, cyclic or acylic, saturated monovalent hydrocarbon, e.g., methyl, ethyl, 1-propyl, 2-propyl, pentyl, and the like.

"Alkylene" means a linear saturated divalent hydrocarbon, or a branched saturated divalent hydrocarbon, e.g., methylene, ethylene, propylene, and the like.

"Aryl" means a monovalent aromatic, such as phenyl, naphthyl and the like.

The PSA systems of the present disclosure generally provide the desired balance of tack, peel adhesion, and shear holding power, and further conform to the Dahlquist criteria, i.e., the modulus of the adhesive at the application temperature, typically room temperature (e.g., about 27 °C), is less than 3x10⁶ dynes/cm at a frequency of 1 Hz.

Some aspects of the present disclosure provide a pressure-sensitive adhesive (PSA). The PSA system can include an antimicrobial polymer having a plurality of pendant groups. The plurality of pendant groups can include a first pendant group comprising a nonpolar compound, a second pendant group comprising a quaternary ammonium compound, and a third pendant group different from the first pendant group or the second pendent group, wherein the third pendant group provides inter-reactivity, such that the antimicrobial polymer can include one or more intermolecular interactions. The antimicrobial polymer can be free of non-ionic hydrophilic moieties. The PSA system can have a glass transition temperature T_{g} ranging from 0 to 50 degrees C.

Other features and aspects of the present disclosure will become apparent by consideration of the detailed description and accompanying drawings.

### BRIEF DESCRIPTION OF THE DRAWINGS

FIG. 1 is a schematic side view of a medical article according to one embodiment of the present disclosure.
FIG. 2 is a schematic side view of a roll of medical tape according to one embodiment of the present disclosure.

### DETAILED DESCRIPTION

Before any embodiments of the present disclosure are explained in detail, it is understood that the invention is not limited in its application to the details of use, construction, and the arrangement of components set forth in the following description or illustrated in the following drawings. The invention is capable of other embodiments and of being practiced or of being carried out in various ways that will become apparent to a person of ordinary skill in the art upon reading the present disclosure. Also, it is understood that the phraseology and terminology used herein is for the purpose of description and should not be regarded as limiting. The use of "including," "comprising," or "having" and variations thereof herein is meant to encompass the items listed thereafter and equivalents thereof as well as additional items. It is understood that other embodiments may be utilized and structural or logical changes may be made without departing from the scope of the present disclosure.

The present disclosure generally relates to pressure-sensitive adhesive systems ("PSA systems") comprising an antimicrobial polymer having a plurality of pendant groups, wherein at least one of the pendant groups comprises an antimicrobial moiety. As a result, the antimicrobial polymer includes a covalently attached, non-leaching antimicrobial.

Generally, "non-leaching" or "non-leachable" antimicrobials refers to antimicrobial materials that are covalently bonded to the chemical structure making up the polymer. Particularly, "non-leaching" means that the quaternary ammonium compounds of the present disclosure do not separate from the antimicrobial polymer or otherwise become non-integral with the polymer under standard uses or exposure conditions.

The PSA system generally includes a glass transition temperature T_{g} ranging from -20 to 50 °C; in some embodiments, ranging from -10 to 40 °C; in some embodiments, ranging from 0 to 30 °C; in some embodiments, from 10 to 30 °C; and in some embodiments, from 20 to 30 °C. In some embodiments, the glass transition temperature T_{g} can be at least -20 °C; in some embodiments, at least -10 °C; in some embodiments, at least 0 °C; in some embodiments, at least 5 °C; in some embodiments, at least 10 °C; and in some embodiments, at least 20 °C. In some embodiments, the glass transition temperature T_{g} can be no greater than 50 °C; in some embodiments, no greater than 40 °C; and in some embodiments, no greater than 30 °C. Said another way, in some embodiments, the PSA system generally includes a glass transition temperature at about room temperature (e.g., about 27 °C) ± 20 °C.

In some embodiments, the PSA system includes the antimicrobial polymer alone, or in combination with suitable tackifiers and/or plasticizers (e.g., excipients), to provide the pressure-sensitive adhesive properties - i.e., one or more glass transition temperatures T_{g} in the above ranges. However, in other embodiments, the PSA system can further include one or more adhesive polymers that are blended with the antimicrobial polymer, in order to give the desired adhesive properties, such that the resulting PSA system comprises a polymer blend. Adhesive polymers that can be employed in the PSA systems of the present disclosure are described in greater detail below.

In some embodiments, the adhesive polymer and the antimicrobial polymer combine to form an interpenetrating network (IPN), such that the PSA system comprises an IPN. An IPN is a polymeric material comprising two or more networks which are at least partially interlaced on a polymer scale but not covalently bonded to each other. In general, an IPN cannot be separated unless chemical bonds are broken.

PSA systems of the present disclosure can be configured to provide acceptable adhesion to skin and to be acceptable for use on skin (e.g., the PSA system should preferably be non-irritating and non-sensitizing). Suitable PSA systems are pressure-sensitive and, in certain embodiments, have a relatively high moisture vapor transmission rate to allow for moisture evaporation. In addition, as mentioned above, PSA systems of the present disclosure can further include one or more additives, such as tackifiers, plasticizers, and rheology modifiers.

In some embodiments, as described in greater detail below, the PSA system of the present disclosure (i.e., whether the PSA includes the antimicrobial polymer alone or in combination with one or more adhesive polymers) can further include an additional (or secondary) antimicrobial agent, e.g., that can be dispersed within the PSA system, as opposed to being covalently bound to, or forming a portion of, the antimicrobial polymer of the PSA system. The antimicrobial polymer, and particularly, one pendant group thereof comprising a quaternary ammonium compound (as described in greater detail below), can be particularly suited for use with dispersed antimicrobial agents, such as cation-based antimicrobial agents (e.g., CHG), because the quaternary ammonium compound of the antimicrobial polymer can function as a compatibilizer for these antimicrobial agents.

### ANTIMICROBIAL POLYMER

The antimicrobial polymer of the PSA systems of the present disclosure can include a plurality of pendant groups, including: (i) a first pendant group comprising a nonpolar compound, (ii) a second pendant group comprising a quaternary ammonium compound, and (iii) a third pendant group different from the first pendant group or the second pendent group. The third pendant group can be particularly suited to provide inter-reactivity within the PSA system. Specifically, as a result of the third pendant group, the antimicrobial polymer can include one or more intermolecular interactions, including but not limited to, covalent bonding, ionic bonding, hydrogen bonding, other suitable intermolecular interations, or a combination thereof. In some embodiments, the third pendant group can also provide reactivity with the adhesive polymer, if employed, such that the PSA system can include one or more intermolecular interactions, including but not limited to, covalent bonding, ionic bonding, hydrogen bonding, other suitable intermolecular interactions, or a combination thereof.

Particularly, in some embodiments, the antimicrobial polymer can include the first pendant group adjacent the second pendant group, the second pendant group adjacent the third pendant group, and the third pendant group adjacent another first pendant group, and so on. However, in other embodiments, the antimicrobial polymer can include a random distribution of the three pendent groups.

Generally, the antimicrobial polymer is free of non-ionic hydrophilic moieties. Particularly, the antimicrobial polymer is free of polyethers (e.g., polyolefinic oxides). As mentioned above, the quaternary ammonium compound of the antimicrobial polymer can function as a compatibilizer for dispersed antimicrobial agents (e.g., cation-based antimicrobial agents, such as CHG), such that the antimicrobial polymer can be free of non-ionic hydrophilic moieties.

The antimicrobial polymers of the present disclosure has the general formula I: 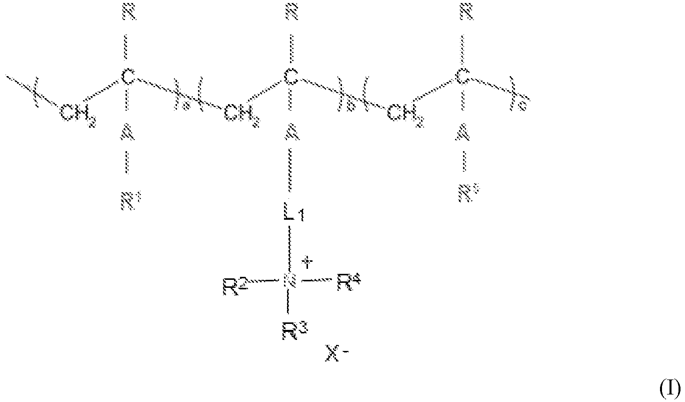 wherein:
each A is a carboxyl group (-COO-), an ester group (-OOC-R-), an oxygen atom (-O-), a urethane group (-O-CO-NH-), a urea group, (-NH-CO-NH-), an alkyl group having from 1 to 12 carbons (i.e., a C1-C12 alkyl), or an aryl group;
R₁ is an alkyl group having from 1 to 22 carbons (i.e., a C1-C22 alkyl), or an aryl group;
L₁ is an alkylene group having from 1 to 3 carbons (i.e., -(CH₂)ₙ-, where n = 1 to 3);
R₂, R₃, and R₄ are each selected from a hydrogen atom (-H), an alkyl group having from 1 to 22 carbons (i.e., a C1-C22 alkyl), or an aryl group;
X is chloride (Cl), a bromide (Br), a tetrafluoroborate (BF₄), an N-fluorobis(trifluoromethanesulfonyl)imide (N(SO₂CF₃)₂), a trifluoromethanesulfonate (OSO₂CF₃), a nonafluorobutanesulfonate (OSO₂C₄F₉), a methylsulfate (OSO₃CH₃), a gluconate (C₆H₁₁O₇), or an acetate (C₂H₃O₂); and
R₅ is a hydrogen atom, N(R₆)₂, Si(OR₆)₃; R₆ is an alkyl group having from 1 to 12 carbons; and a + b + c is at least 50.

In some embodiments, the term "polymer" can refer to a molecular chain of at least 50 repeat units, in some embodiments, at least 100 repeat units, in some embodiments, at least 200 repeat units, in some embodiments, at least 500 repeat units, in some embodiments, at least 1000 repeat units, in some embodiments, at least 5000 repeat units, and in some embodiments, at least 10,000 repeat units.

As a result, the sum of a, b and c in Formula I above is at least 50, in some embodiments, at least 100, in some embodiments, at least 200, in some embodiments, at least 500, in some embodiments, at least 1000, in some embodiments, at least 5000, and in some embodiments, at least 10,000.

As shown in Formula I above, the first pendant group of the antimicrobial polymer can include a non-polar compound, and can have the general Formula II: 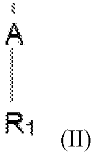 where A and R₁ have the definitions given above with respect to Formula I.

Examples of specific first pendant groups that can be employed include, but are not limited to, any of butyl, octyl, decyl, lauryl, bornyl, and octyldecyl.

As shown in Formula I above, the second pendant group of the antimicrobial polymer can include a quaternary ammonium compound, and can have the general Formula III: 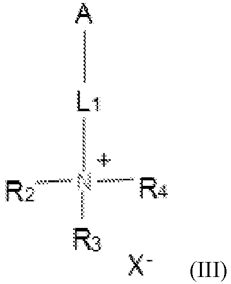 where A, L₁, R₂, R₃, R₄ and X- have the definitions given above with respect to Formula I.

Examples of specific second pendant groups of the present disclosure (e.g., the quaternary ammonium compounds that can be used to form at least a portion of the second pendant groups) are described in greater detail below.

As a result, as shown in Formula I above, in some embodiments, the third pendant group of the antimicrobial polymer can have the general Formula IV: 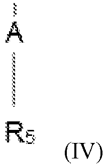
where A has the definition given above with respect to Formula I;
R₅ is a hydrogen atom H, N(R₆)₂, Si(OR₆)₃; and
R₆ is an alkyl group having from 1 to 12 carbons (i.e., a C1-C12 alkyl).

In some embodiments, the antimicrobial polymer can be derived from a first monomer, a second monomer, and a third monomer. In some embodiments, these monomers may have the structures shown in formulas (V) through (VII):

Z - R¹ (V)

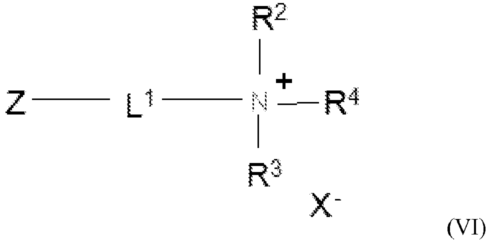

Z - R⁵ (VII)

where L₁, R₁, R2, R₃, R₄, R₅, and X- have the definitions given above with respect to Formula I.

In some embodiments, Z in Formulas (V), (VI) and (VII) can be independently selected from, and have the reactional moiety of, an acrylate, as shown in Formula (VIII); a vinyl ether, as shown in Formula (IX); a vinyl urethane, as shown in Formula (X); a vinyl urea, as shown in Formula (XI), or other suitable moieties. 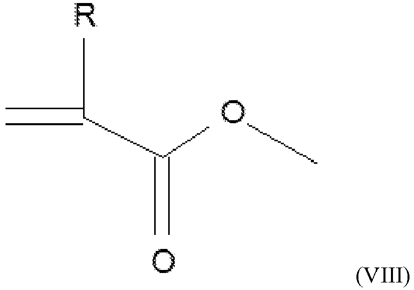 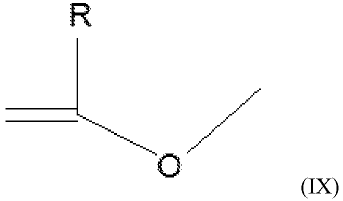 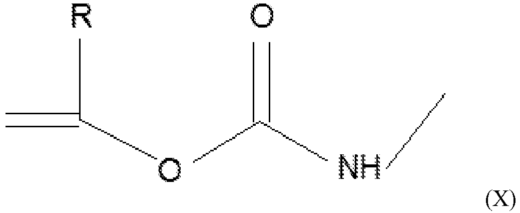 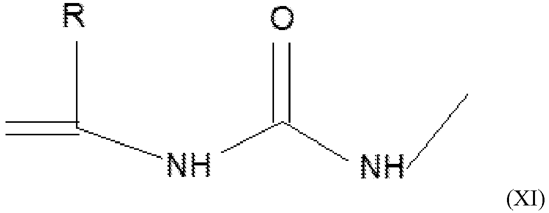 where R in Formulas (VIII) - (XI) represents the remainder of the monomer of interest.

In some embodiments, the antimicrobial polymer of the present disclosure can be a polyacrylate. In such embodiments, the antimicrobial polymer can be derived from acrylate monomers, such as the quaternary ammonium compound (i.e., that will form the second pendant group) of the antimicrobial polymer) shown in Formula XII: 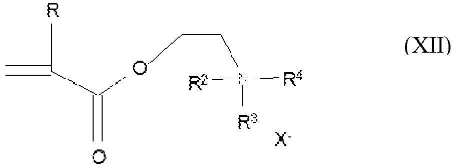 where:
R is selected from H and CH₃; and
R₂, R₃, R₄ and X- have the definitions given above with respect to Formula I.

In some embodiments, at least one of R₂, R₃, and R₄ can be selected from C₄H₉, C₆H₁₃, C₁₀H₂₁, C₁₂H₂₅, C₁₆H₃₃, C₁₈H₃₇, C₂₀H₄₁, and C₂₂H₄₅. In some embodiments, particularly suitable values for "n" can range from 4 to 16, in some embodiments, from 6 to 16, in some embodiments, from 10 to 16, and in some embodiments, from 10 to 12. Suitable examples of quaternary amine-functionalized ethylenically unsaturated monomers that can form the quaternary ammonium compound (i.e., that can form at least a portion of the second pendant group) can include dimethyldecylammoniumethylacrylate (DMAEA-C₁₀), dimethyldecylammoniumethylmethacrylate (DMAEMA-C₁₀), dimethylhexadecylammoniumethylacrylate (DMAEA-C₁₆), and dimethylhexadecylammoniumethylmethacrylate (DMAEMA-C₁₆).

Specific examples of suitable quaternary amine-functionalized ethylenically unsaturated monomers that can form the quaternary ammonium compound can include dimethylhexadecylammoniumethylacrylate halides (DMAEA-C₁₆ halides; e.g., dimethylhexadecylammoniumethylacrylate bromides (DMAEA-C₁₆Br)); dimethyldecylammoniumethylacrylate halides (DMAEA-C₁₀ halides; e.g., dimethyldecylammoniumethylacrylate bromides (DMAEA-C₁₀Br)), dimethylhexadecylammoniumethylmethacrylate halides (DMAEMA-C₁₆ halides; e.g., dimethylhexadecylammoniumethylmethacrylate bromides (DMAEMA-C₁₆Br)); dimethyldecylammoniumethylmethacrylate halides (DMAEMA-C₁₀ halides; e.g., dimethyldecylammoniumethylmethacrylate bromides (DMAEMA-C₁₀Br)) and derivatives thereof.

Examples of suitable DMAEA-Cₙ halides and DMAEMA-Cₙ halides include derivatives of DMAEA-C₁₆Br and DMAEMA-C₁₆Br, as described below, but it should be understood that similar derivatives of other DMAEA-Cₙ halides and DMAEMA-Cₙ halides are within the spirit and scope of the present disclosure, and one of ordinary skill in the art would understand how to extend the description below regarding the formation of DMAEA-C₁₆Br and DMAEMA-C₁₆Br to such other halides.

Suitable DMAEMA derivatives (e.g., DMAEMA-CₙBr) have the structural formula of Formula XIII: 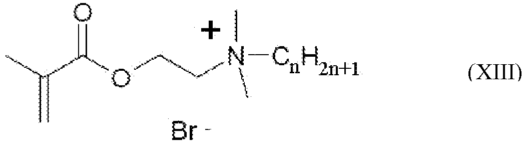 where suitable values for "n" range from 2-22 with particularly suitable values for "n" ranging from 4-16, in some embodiments, from 6-16, in some embodiments, from 10-16, and in some embodiments, from 10-12. Such polymer-chain lengths allow the DMAEMA derivative to move enough within the PSA system while also preventing the DMAEMA derivative from phase separating from the resulting PSA system.

By way of example only, DMAEMA-C₁₆Br and its derivatives may be formed by combining dimethylaminoethylmethacrylate salt, acetone, 1-bromohexadecane, and optionally, an antioxidant. The mixture may be stirred for about 16 hours at about 35°C and then allowed to cool to room temperature. The resulting white solid precipitate may then be isolated by filtration, washed with cold ethyl acetate, and dried under vacuum at 40°C.

Similarly, DMAEA-C₁₆Br and its derivatives may be formed by combining dimethylaminoethylacrylate, of acetone, 1-bromohexadecane, and optionally, an antioxidant. The mixture may be stirred for 24 hours at 35°C, and then allowed to cool to room temperature. The acetone may then be removed by rotary evaporation under vacuum at 40°C. The resulting solids may then be washed with cold ethyl acetate and dried under vacuum at 40°C.

Other DMAEMA-CₙBr or DMAEA- CₙBr based monomers were made following the same procedure outlined above where n varied between 4 and 22. Other DMAEMA-CₙX based monomers can be made using an ion exchange reaction.

The alkyl chain length of the quaternary ammonium compound that forms at least a portion of the second pendant group can cause a change in both conformation and charge density of the antimicrobial polymer, which in turn can influence the mode of interaction with a cytoplasmic membrane. For the quaternary ammonium compounds of the present disclosure, a hydrophilic-lipophilic balance can influence the antimicrobial properties. As a result, in some embodiments, the antimicrobial activity of quaternary ammonium compounds can increase with increasing alkyl chain length (i.e, increasing hydrophobicity), in some cases, up to about n=10 (i.e., C₁₀H₂₁), after which a decrease in antimicrobial activity can occur with an increase in chain length (i.e., decreasing hydrophilicity). Also, excessive hydrophobicity can give rise to nonspecific binding, which is some cases may result in lysis of human red blood cells.

### ANTIMICROBIAL PERFORMANCE

The PSA systems of the present disclosure are particularly suited to reduce microbial growth. As a result, the PSA systems of the present disclosure include sufficient antimicrobial material to substantially inhibit microbial growth. In some embodiments, substantially inhibiting microbial growth can include exhibiting at least a 1 log reduction, in some embodiments, at least a 2 log reduction, in some embodiments, at least a 3 log reduction, in some embodiments, at least a 5 log reduction, and in some embodiments, at least a 6 log reduction in either gram positive or gram negative bacteria, particularly at 24 hours, e.g., when tested pursuant to JIS Z 2801 (Japan Industrial Standards; Japanese Standards Association; Tokyo, JP; modified as described in the Examples below), and compared to an untreated/uncoated control, as exhibited in the Examples below.

In some embodiments, substantially inhibiting microbial growth can include exhibiting a zone of inhibition that is no greater than (or matches) the sample size (or area) employed, e.g., when tested pursuant the Zone of Inhibition test described in the Examples below. In some embodiments, substantially inhibiting microbial growth can include exhibiting a zone of inhibition that is greater than the sample size employed. PSA systems of the present disclosure, for example, may achieve a zone of inhibition that is greater than the sample size when an additional antimicrobial agent is dispersed within the PSA system of the present disclosure. Examples of antimicrobial agents that can be dispersed within the PSA system of the present disclosure are described in greater detail below.

In some embodiments, sufficient antimicrobial moiety(ies) in the PSA systems can include at least (or more than) 0.1 parts by weight of quaternary ammonium compound (i.e., by virtue of the second pendant group in the antimicrobial polymer) per 100 parts by dry weight of the PSA system (0.1 wt%); in some embodiments, at least (or more than) 0.25 wt% of quaternary ammonium compound; in some embodiments, at least (or more than) 0.5 wt% of quaternary ammonium compound; in some embodiments, at least (or more than) 1 wt% of quaternary ammonium compound; in some embodiments, at least (or more than) 2 wt% of quaternary ammonium compound; in some embodiments, at least (or more than) 5 wt% of quaternary ammonium compound; in some embodiments, at least (or more than) 10 wt% of quaternary ammonium compound; in some embodiments, at least (or more than) 20 wt% of quaternary ammonium compound; and in some embodiments, at least (or more than) 25 wt% of quaternary ammonium compound.

In some embodiments, sufficient antimicrobial material in the PSA systems of the present disclosure can include from 0.1 wt% to 25 wt% of quaternary ammonium compound; and in some embodiments, from 2 wt% to 5 wt% of quaternary ammonium compound.

### ADHESIVE POLYMERS

In general, adhesive polymers, if employed, can be chosen that give the PSA system sufficient adhesive properties, as well as the additional properties described above, such as being non-irritating and non-sensitizing, and having a relatively high moisture vapor transmission rate.

In some embodiments, the adhesive polymer (if employed) can include at least one of an acrylate polymer, a silicone polymer, a urethane polymer, a synthetic rubber, a natural rubber, a polyolefin, and a combination thereof.

In some embodiments employing the adhesive polymer, the adhesive polymer can be present in a concentration of at least 30 wt%, based on the total weight of the PSA system; in some embodiments, at least 60 wt%; and in some embodiments, at least 80wt%.

### METHODS OF MAKING

In general, the PSA systems of the present disclosure can be formed by preparing an antimicrobial polymer of the present disclosure that includes a glass transition temperature T_{g} that meets the above-described limitations.

Alternatively, the PSA systems of the present disclosure can be formed by blending at least one antimicrobial polymer with at least one adhesive polymer. The resulting PSA system can have the desired pressure-sensitive adhesive properties as a result of the antimicrobial polymer used; a tackifier used; a plasticizer used; and/or an adhesive polymer used.

In some embodiments, hotmelt antimicrobial PSA systems of the present disclosure can be prepared by co-extruding hotmelt adhesives (e.g. 95/5 Iso-octyl acrylate /2-hycrosyethyl acrylate (IOA/2-HEA)) with an antimicrobial polymer (e.g. AM-1 of the Examples below) through a twin screw extruder. In some embodiments, a tackifier and/or a plasticizer can be to the mixture during co-extrusion. The hotmelt processed antimicrobial PSA systems can be direct coated onto a backing (e.g., of a medical article, as described below) or a release liner (e.g., any of those described below), and can optionally be subjected to ultraviolet (UV) or electron beam (e-beam) radiation for further crosslinking.

### TACKIFIERS AND PLASTICIZERS

The PSA systems prepared from the compositions of this disclosure provide the desired balance of tack, peel adhesion, and shear holding power.

As mentioned above, in some embodiments, the PSA system of the present disclosure can include a tackifier, a plasticizer, or the like, or combinations thereof.

A tackifier can be added to modify the rheology properties of PSA systems. A tackifier can also modify properties such as tack, i.e., the ability to stick with finger pressure; adhesion, i.e., the binding force of the adhesive; and/or cohesive strength (i.e., the ability of adhesive polymers to remove cleanly from the substrate, without leaving adhesive residue on the substrate. The proper tackifier can also lend the adhesive composition sufficient viscous flow properties to bring the adhesive and substrate into intimate contact, thus allowing adhesive bonds to form.

In some embodiments, the tackifier useful for acrylic adhesive polymersof the present disclosure can include, but is not limited to hydrogenated, aromatic hydrocarbon tackifying resin.

In some embodiments, the tackifier useful for rubber based adhesive polymers of the present disclosure can include, but is not limited to coumarone indenes, rosin esters, aromatic resins, mixed aromatic/aliphatic resins, aromatic modified hydrocarbon resins, liquid hydrocarbon resins, liquid polyterpenes, liquid rosin esters, and liquid polystyrene resins.

In some embodiments, the tackifier useful for silicone adhesive polymers of the present disclosure can include, but is not limited to a silicate tackifier resin, known as an "MQ resin". MQ silicate resins useful in the present adhesive composition include those composed of the structural units M, D, T, Q, and combinations thereof. For example, MQ silicate resins, MQD silicate resins, and MQT silicate resins that also may be referred to as copolymeric silicate resins and that preferably have a number average molecular weight of about 100 to about 50,000, more preferably about 500 to about 10,000 and generally have methyl substituents. Silicate resins include both nonfunctional and functional resins, the functional resins having one or more functionalities including, for example, silicon-bonded hydrogen, silicon- bonded alkenyl, and silanol.

Plasticizers are generally added to soften a material by either softening the final product or to increase the workability of the material before it hardens. Generally, plasticizers work by embedding themselves between the chains of polymers, spacing them apart or increasing the free volume, and thus lowering the glass transition temperature which makes the material more pliant.

In some embodiments, the plasticizers of the present disclosure can include, but are not limited to polyethylene glycol, polypropylene glycol, esters of polyethylene glycol and polypropylene glycol, glycerin, polypropylene glycol-polyethylene oxide copolymer, block copolymers of ethylene oxide and propylene oxide, polyethylene oxide alkylphenyl esters, sorbitol, mannitol, lanolin, lecithin, silicone oil, silicone gum or combinations thereof.

### RHEOLOGY MODIFIERS

Rheology modifiers are sometimes referred to as thickeners. Suitable rheology modifiers that can be employed in the PSA systems include, but are not limited polyurethanes, acrylic polymers, latex, styrene/butadiene, polyvinylalcohol, clay, cellulosics, proteins, and organic silicones. Specific examples of rheology modifiers that can be employed include, but are not limited to, those available under the trade designations THIXATROL®, THIXCIN®, RHEOLATE®, or combinations thereof.

### ANTIMICROBIAL AGENT

In some embodiments, the PSA system of the present disclosure can include an additional (or secondary) antimicrobial agent, i.e., that is dispersed within the PSA system, compared to the quaternary ammonium compound that is covalently bound to the antimicrobial polymer via the second pendant group, as described above.

In some embodiments, the antimicrobial agent can include, but is not limited to iodine, complexed forms of iodine, chlorhexidine salts, parachlorometaxylenol, triclosan, hexachlorophene, fatty acid esters, phenols, surfactants having a C12-C22 hydrophobe and a quaternary ammonium group, quaternary ammonium salts, quaternary ammonium silanes, hydrogen peroxide, silver, silver salts, silver oxide, silver sulfadiazine, or combinations thereof.

In some embodiments, quaternary ammonium salts can include, but are not limited to, chlorhexidine gluconate (CHG), chlorhexidine digluconate (CHD), polyhexamethylene biguanides (PHMB), benzalkonium (e.g., benzalkonium chloride), benzethonium, polyDADMAC, quaternary ammonium compounds, homopolymers or copolymers comprising pendant quaternized ammonium moieties, or combinations thereof.

In some embodiments, the PSA system can include no greater than 5 wt% of the additional (i.e., dispersed) antimicrobial agent, based on the total weight of the PSA system; in some embodiments, no greater than 4 wt%; in some embodiments, no greater than 3 wt%; in some embodiments, no greater than 2 wt%; and in some embodiments, no greater than 1 wt%.

### MEDICAL ARTICLES

In some embodiments, the PSA systems of the present disclosure can be used in medical articles. As used herein, "medical articles" include, but are not limited to, medical tapes, skin closure tapes, surgical drapes, medical dressings (e.g., intravenous dressings and wound dressings), electrodes, wearable sensors, ostomy pouches, adhesive patches, medical tubing securement devices, first aid bandages, and combinations thereof.

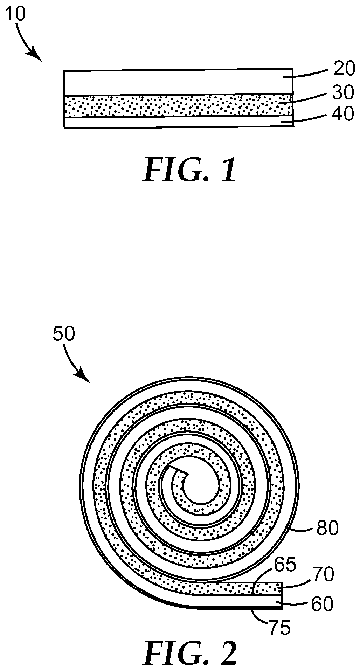

FIG. 1 illustrates a medical article 10 according to one embodiment of the present disclosure. As shown in FIG. 1, the medical article 10 can include a medical backing 20, a PSA system 30 of the present disclosure, and a release liner 40 in contact with the adhesive to prevent premature adhesion.

The medical backing can include single-layer and multi-layer constructions. Useful backings can include, e.g., a polymeric foam layer, a polymeric film layer, a fabric layer, a nonwoven layer, and combinations thereof, provided such backings have the proper breathable, optical, and mechanical properties.

Potentially useful polymeric backing materials are disclosed in U.S. Patent No. 5,516,581 (Kreckel et al.) and PCT Publication No. WO95/06691. Representative examples of potentially useful polymeric backing materials for polymeric foam layers or solid polymeric film layers include polyurethane; polyolefins, e.g., polyethylene, including high density polyethylene, low density polyethylene, linear low density polyethylene, and linear ultra low density polyethylene, polypropylene and polybutylenes; vinyl copolymers, e.g., polyvinyl chlorides, both plasticized and unplasticized, and polyvinyl acetates; olefin copolymers, e.g., ethylene/methacrylate copolymers, ethylene/vinylacetate copolymers, acrylonitrile-butadiene-styrene copolymers, and ethylene/propylene copolymers; acrylic polymers and copolymer; and combinations thereof. Mixtures or blends of any plastic or plastic and elastomer materials, such as polypropylene/polyethylene, polyurethane/polyolefin, polyurethane/polycarbonate, and polyurethane/polyester, can also be used.

Polymeric foams can be selected to optimize tape properties such as conformability and resiliency, which are useful when the tape is to be adhered to surfaces having surface irregularities, e.g., painted wallboard. Conformable and resilient polymeric foams are well suited for applications in which the adhesive tape is to be adhered to surfaces having surface irregularities. Such is the case with a typical wall surface. Polymeric foam layers for use in the backing generally will have a density of about 2 to about 30 pounds per cubic foot (about 32 to about 481 kg/m³), particularly in tape constructions where the foam is to be stretched to effect debonding. Where only one polymeric film or foam layer of a multi-layer backing is intended to be stretched to effect debonding, that layer should exhibit sufficient physical properties and be of a sufficient thickness to achieve that objective.

Polymeric films may be used to increase load bearing strength and rupture strength of the tape. Films are particularly well suited to applications involving adhering smooth surfaces together. A polymeric film layer typically has a thickness of about 10 micrometers (0.4 mil) to about 254 micrometers (10 mils).

In some embodiments, the backing can include an elastomeric material. Suitable elastomeric backing materials include, e.g., styrene-butadiene copolymer, polychloroprene (i.e., neoprene), nitrile rubber, butyl rubber, polysulfide rubber, cis-1,4-polyisoprene, ethylenepropylene terpolymers (e.g., EPDM rubber), silicone rubber, silicone elastomers such as silicone polyurea block copolymers, polyurethane rubber, polyisobutylene, natural rubber, acrylate rubber, thermoplastic rubbers, e.g., styrene-butadiene block copolymers and styrene- isoprene-styrene block copolymers, and thermoplastic polyolefin rubber materials.

FIG. 2 illustrates a medical tape 50, shown in rolled form, according to one embodiment of the present disclosure. As shown in FIG. 2, the medical tape 50 can include a medical backing 60 (such as those described above with respect to the medical backing 20 of FIG. 1); a PSA system 70 of the present disclosure on a first surface 65 of the backing 60, and a low adhesion backsize coating 80 on a second (i.e., opposite) surface 75 of the backing 60.

When the PSA system 70 is coated onto one side of the backing 60, a release coating (e.g., a low adhesion backsize (LAB) coating) can optionally be coated on the opposite face to allow the resultant tape to unwind from itself when wound in a roll or to release when in a pad form. When utilized, the release coating composition should be compatible with the adhesive composition and not degrade the adhesive properties of the tape, such as by being transferred to the adhesive composition.

Release coating compositions for the liner or LAB layer of tapes may include silicone, alkyl, or fluorochemical constituents, or combinations thereof, as the release imparting component. Useful release coating compositions for the invention include silicone containing polymers, such as silicone polyurethanes, silicone polyureas and silicone polyurethane/ureas, such as those described in U.S. Pat. Nos. 5,214,119, 5,290,615, 5,750,630, and 5,356,706, and silicone acrylate grafted copolymers described in U.S. Pat. Nos. 5,032,460, 5,202,190, and 4,728,571. Other useful release coating compositions include fluorochemical containing polymers such as those described in U.S. Pat. No. 3,318,852, and polymers containing long alkyl side chains such as polyvinyl N-alkyl carbamates (e.g., polyvinyl N- octadecyl carbamates) as described in U.S. Pat. No. 2,532,011, and copolymers containing higher alkyl acrylates (e.g., octadecyl acrylate or behenyl acrylate), such as those described in U.S. Pat. No. 2,607,711, or alkyl methacrylates (e.g., stearyl methacrylate) such as those described in U.S. Pat. Nos. 3,502,497 and 4,241,198, where the alkyl side chain includes from about 16 to 22 carbon atoms.

The embodiments described above and illustrated in the figures are presented by way of example only and are not intended as a limitation upon the concepts and principles of the present disclosure. As such, it will be appreciated by one having ordinary skill in the art that various changes in the elements and their configuration and arrangement are possible without departing from the spirit and scope of the present disclosure.

All references and publications cited herein are expressly incorporated herein by reference in their entirety into this disclosure.

The following embodiments are intended to be illustrative of the present disclosure and not limiting.

The following working examples are intended to be illustrative of the present disclosure and not limiting.

### EXAMPLES

### Materials

Materials used for the examples are shown in Table 1.

**Table 1: Materials List**

| Material | **Description** | **Source** |
|---|---|---|
| 1 -Bromohexadecane | 1 -Bromohexadecane | Chemtura Corporation, Bay Minette, AL |
| DMAEMA | 2-(Dimethylamino)ethyl methacrylate | CIBA, Marietta, GA |
| NIPAAM | N-Isopropylacrylamide | TCI, Portland, OR |
| Tegopren 5840 | Tegopren 5840 | Evonik Corporation, Hopewell, VA |
| Acrysol RM-8W | Acrysol RM-8W | Dow, Philadelphia, PA |
| ACM | Acrylamide | Sigma-Aldrich; St. Louis, MO |
| t-Octyl ACM | t-Octylacrylamide | Polyscience Inc., Warrington, PA |
| HEA | Hydroxyethyl acrylate | Sigma-Aldrich; St. Louis, MO |
| HEMA | Hydroxyethyl methacrylate | Sigma-Aldrich; St. Louis, MO |
| BHT | 2,6-ditert-butyl-4-methylphenol | Sigma-Aldrich, St. Loius, MO |
| EQ-25 | ETHOQUAD® C-25 | Akzo Nobel N.V. of Amsterdam, the Netherlands |
| MEHQ | 4-Methoxyphenol | Alfa Aesar, Ward Hill, MA |
| pDADMAC | Polydiallyldimethylammonium chloride | Sigma-Aldrich, St. Loius, MO |
| DMAEA-MCl | Dimethylaminoethyl acrylate methyl chloride | CIBA, Marietta, GA |
| A-174 | Methacryloylpropyl trimethoxy silane | Sigma-Aldrich, St. Loius, MO |
| MAA | Methacrylic Acid | Alfa Aesar, Ward Hill, MA |
| VAc | Vinyl Acetate | Dow, Midland, MI |
| V-50 | 2,2'-Azobis(2-methylpropionamidine)dihydrochloride | duPont deNemours and Co, Wilmington, DE |
| IOA | Iso-octyl acrylate, CAS # 29590-42-9 | Sartomer USA, LLC; Exton, PA |
| Vazo™-67 | 2,2-Azobis(2-methylbutyronitrile) | Dupont, Wilmington, DE |
| CHG | Chlorhexidine gluconate, 20% aqueous solution | Xttrium Laboratories, Inc., Chicago, IL |
| PET film | Scotch Pak™ polyester film (3.85 mil) | 3M Company, St. Paul, MN |
| PU film | Estane® 58309 polyurethane film (0.9 mil) | Lubrizol, Wickliffe, OH |
| *S. aureus* | *Staphylococcus aureus,* ATCC# 6538 | Microbiologics, Inc., St. Cloud, MN |
| *P. aeruginosa* | *Pseudomonas aeruginosa,* ATCC# 9027 | Microbiologics, Inc., St. Cloud, MN |

### Test Methods

### Microbial Kill

Microbial kill (log reduction) was determined based on test method JIS Z 2801 (Japan Industrial Standards; Japanese Standards Association; Tokyo, JP). A bacterial inoculum was prepared in a solution of 1 part nutrient broth (NB) and 499 parts phosphate buffer. A portion of the bacterial suspension (150ul) was placed onto the surface of a test sample and the inoculated sample was incubated up to 24 hours at 37+/-1 °C. After incubation, the test sample was placed into 20ml of D/E (Dey/Engley) Neutralizing Broth (Difco™, BD, San Jose, CA). The number of surviving bacteria in the Neutralizing Broth was determined by using Petrifilm™ AC plates, (3M Company, St. Paul, MN). The samples were serially diluted by ten-fold dilutions and each dilution was plated onto the Petrifilm™ plate. Plates were incubated for 48 hours at 37+/-1°C. After incubation, the colonies were counted and the number of surviving bacteria were calculated. Microbial kill was determined by subtracting the log of the number of surviving colonies on the test samples from the log of the number of surviving colonies on a polyester control film to achieve the microbial log reduction, as reported in Table 4.

For examples E-19-E-25, the microbial kill was determined using modified ASTM E 2315-03 (Reapproved 2008). The modifications included the use of TSB (Tryptone Soya Broth) instead of PBW (phosphate buffered water), and the use of Petrifilm™ rather than TSA.

### Zone of Inhibition

The zone of inhibition (in mm) was determined based on the Kirby-Bauer disk diffusion susceptibility test. The test microorganisms were grown tryptic soy agar (BD Difco™, Franklin Lakes, NJ) for 24 hours at 37+/-1 °C to obtain fresh growth. A bacterial inoculum was prepared for each test organism by re-suspending fresh growth in phosphate buffer equal to 0.5 McFarland Standard. This inoculum was swabbed onto Mueller-Hinton agar (Teknova, Hollister, CA) to obtain a lawn of bacteria. A 7 mm sample disk was placed on top of the agar and the plates were incubated for 24 hours at 37+/-1°C. After incubation, the total zone of inhibited growth (including the sample disk) was measured. A value greater than 7 mm indicated inhibition extended beyond the disk, while a value of 0 mm indicated there was no zone of inhibition, either under the sample disk or extended from the sample disk (bacterial growth was observed under and around the disk).

For examples E-19-E-25, a 1 inch (25.4 mm) disk rather than a 7 mm disk was used.

### Peel Adhesion

A 2.54-cm (1-inch) wide strip of antimicrobial adhesive tape was laminated onto a stainless steel (SS) panel using a 2.0 kg (4.5 lb) rubber roller to give a bonded article. After a dwell time of 20 minutes at 23 °C and 50% relative humidity (RH), a 180 degree angle peel test was performed using a Model 3M90 Slip/Peel Tester (Instrumentors, Inc., Strongville, OH) at 30.5 cm/min. (12 in./min), with data collected and averaged over 10 seconds, according to the test method ASTM Designation D3330/D330M-04. The peel in g/cm was reported.

### Examples

### Antimicrobial Polymer Synthesis

### DMAEMA-C₁₆Br/IOA/A-174 (AM-1)

A clean reactor fitted with over head condenser, mechanical stirrer, and temperature probe was charged with 918 parts of acetone, 807 parts of 1-bromohexadecane, 415.5 parts of DMAEMA, 2.0 parts of BHT, and 2.0 parts of MEHQ. The batch was stirred at 150 rpm and 90/10 O₂/N₂ was purged through the solution. The mixture was heated to 74°C for 18 hours followed by addition of 918 parts EtOAc with stirring at high speed. Heat was removed and the solution was allowed to cool to room temperature. The precipitated white solid was isolated by filtration and washed with 200 parts of cold EtOAc and dried in a vacuum oven at 40° C for 8 hours. This was the DMAEMA-C₁₆Br monomer.

In a clean reaction bottle 50 parts of DMAEMA-C₁₆Br monomer, 40 parts of IOA, 10 parts A-174, 0.5 parts of Vazo™-67, and 300 parts of IPA were added. The mixture was purged with nitrogen for 3 minutes. The reaction bottle was sealed and heated in a 65°C preheated water bath with mixing for 17 hours. Additional Vazo™-67 was added (0.1 parts) and the solution heated with mixing for 8 hours to produce the DMAEMA-C₁₆Br/IOA/A-174 polymer (25% solids in IPA). This was Antimicrobial-1 (AM-1).

### pDADMAC (AM-2) - Comparative

pDADMAC was used as AM-2 as a comparative antimicrobial polymer. As a result, Examples 4 and 9-11 below represent comparative examples.

### DMAEA-MCl/IOA/VAc/MAA (AM-3)

In a clean reaction bottle 8 parts of DMAEA-MCl, 85 parts of IOA, 5 parts of VAc, 2 parts of MAA, 0.5 parts of V-50, and 100 parts of water were taken. The mixture was purged with nitrogen for 3 minutes. The reaction bottle was sealed and placed in a 50°C preheated water bath with mixing mechanism. The reaction mixture was heated for 17 hours at 50°C with mixing. To convert the residual monomer to >99.5%, another 0.1 parts of V-50 was added, the solution was purged and sealed. The bottle was placed in the 50°C water bath with mixing mechanism and heated for 8 hours.

### AM-4 through AM-20

AM-4 through AM-20 were prepared as described for AM-3, with the components listed in Table 2.

**Table 2: Antimicrobial Polymer Compositions**

| **AM** | **DMAEA -MCl** | **IOA** | **VAC** | **NIPAAM** | **ACM** | **t-Octyl ACM** | **HEA** | **HEMA** | **EQ-C25** | **V-50** | **Water** |
|---|---|---|---|---|---|---|---|---|---|---|---|
| **4** | **4** | **91** | **0** | **5** | **0** | **0** | **0** | **0** | **1** | **0.5** | **300** |
| **5** | **8** | **87** | **0** | **5** | **0** | **0** | **0** | **0** | **1** | **0.5** | **150** |
| **6** | **10** | **85** | **0** | **5** | **0** | **0** | **0** | **0** | **1** | **0.5** | **200** |
| **7** | **8** | **90** | **0** | **0** | **2** | **0** | **0** | **0** | **1** | **0.375** | **100** |
| **8** | **8** | **85** | **4** | **3** | **0** | **0** | **0** | **0** | **1** | **0.375** | **100** |
| **9** | **8** | **85** | **4** | **3** | **0** | **0** | **0** | **0** | **1** | **0.375** | **300** |
| **10** | **8** | **87** | **2** | **3** | **0** | **0** | **0** | **0** | **1** | **0.375** | **300** |
| **11** | **8** | **85** | **2** | **5** | **0** | **0** | **0** | **0** | **1** | **0.375** | **300** |
| **12** | **8** | **88** | **2** | **0** | **2** | **0** | **0** | **0** | **1** | **0.375** | **300** |
| **13** | **8** | **87** | **2** | **0** | **3** | **0** | **0** | **0** | **1** | **0.375** | **300** |
| **14** | **8** | **87** | **2** | **3** | **0** | **0** | **0** | **0** | **1** | **0.375** | **300** |
| **15** | **8** | **85** | **2** | **5** | **0** | **0** | **0** | **0** | **1** | **0.375** | **300** |
| **16** | **8** | **80** | **2** | **0** | **0** | **5** | **0** | **5** | **1** | **0.375** | **300** |
| **17** | **8** | **82** | **5** | **0** | **0** | **5** | **0** | **0** | **1** | **0.375** | **300** |
| **18** | **8** | **85** | **2** | **0** | **0** | **5** | **0** | **0** | **1** | **0.375** | **400** |
| **19** | **8** | **77** | **5** | **0** | **0** | **5** | **5** | **0** | **1** | **0.375** | **400** |
| **20** | **8** | **82** | **5** | **0** | **0** | **0** | **3** | **0** | **1** | **0.375** | |

### Adhesive Preparation

Table 3 lists the adhesives utilized in the example preparations.

**Table 3: Adhesives**

| **Adhesive** | **Description** |
|---|---|
| A-1 | IOA/Acrylamide, 97/3 (see RE24906) |
| A-2 | IOA/Acrylic acid/Ethylene oxide acrylate, 70/15/15 with 1% polyethyl oxazoline (see Example #104 in US4737410) |
| A-3 | Silicone adhesive (see Example #12 in WO2010056544) |
| A-4 | IOA/ACM/Vac, 71/9/20 (see "Preparation of Isooctyl Acrylate/Acrylamide/Vinyl Acetate 73/7/20 Copolymer" in US6132760) |

### Example-1 (E-1)

Adhesive A-1 (100 parts, by weight) was mixed with antimicrobial AM-1 (10 parts) on a roller mill for 24 hours. This mixture was then coated on PET film with a knife blade at 50 microns thick and dried at 66 °C for 10 minutes to form Example-1 (E-1).

Examples E-2 through E-17 were prepared in a similar manner. Compositions are shown in Table 4, and test results in Tables 5-8.

Example 18 (E-18) did not include an adhesive polymer (i.e., represents a self-adhesive antimicrobial polymer of the present disclosure), as shown in Table 4. E-18 was also coated on PET film, as described above with respect to E-1. E-18 was further tested for peel adhesion (on stainless steel (SS)) and microbial kill, at various levels of aging (Tables 6 and 7), demonstrating stable microbial kill and stable adhesion performance.

Examples 19-25 (E-19 - E-25) also did not include an adhesive polymer (i.e., represent a self-adhesive antimicrobial polymer of the present disclosure), as shown in Table 4. CHG, i.e., a secondary antimicrobial agent, was added to the antimicrobial polymer, in the amounts shown in Table 4. Examples E-19 - E-25 were each coated on PU film with a knife blade at about 40 microns thick and dried at 66 °C for 10 minutes. Compositions are shown in Table 4, and test results in Table 8.

**Table 4: Example Antimicrobial Adhesive Formulations**

| **Example** | **Antimicrobial Polymer** | | **Adhesive Polymer** | | **Secondary Antimicrobial** | |
|---|---|---|---|---|---|---|
| | **Composition** | **Parts** | **Composition** | **Parts** | **Composition** | **Parts** |
| E-1 | AM-1 | 10 | A-1 | 100 | none | |
| E-2 | AM-1 | 20 | A-1 | 100 | none | |
| E-3 | AM-1 | 5 | A-1 | 100 | none | |
| E-4 | AM-2 | 5 | A-1 | 100 | none | |
| E-5 | AM-1 | 5 | A-1 | 100 | none | |
| E-6 | AM-1 | 15 | A-2 | 100 | none | |
| E-7 | AM-1 | 30 | A-2 | 100 | none | |
| E-8 | AM-1 | 5 | A-2 | 100 | none | |
| E-9 | AM-2 | 15 | A-2 | 100 | none | |
| E-10 | AM-2 | 30 | A-2 | 100 | none | |
| E-11 | AM-2 | 5 | A-2 | 100 | none | |
| E-12 | AM-1 | 15 | A-2 | 100 | none | |
| E-13 | AM-1 | 5 | A-2 | 100 | none | |
| E-14 | AM-1[a] | 10 | A-3 | 100 | none | |
| E-15 | AM-1 | 5 | A-4 | 100 | none | |
| E-16 | AM-1 | 15 | A-4 | 100 | none | |
| E-17 | AM-1 | 30 | A-4 | 100 | none | |
| E-18 | AM-3 | 100 | none | | none | |
| E-19 | AM-4 | 100 | none | | CHG | 0.01 |
| E-20 | AM-4 | 100 | none | | CHG | 0.2 |
| E-21 | AM-5 | 100 | none | | CHG | 0.01 |
| E-22 | AM-5 | 100 | none | | CHG | 0.1 |
| E-23 | AM-6 | 100 | none | | CHG | 0.01 |
| E-24 | AM-6 | 100 | none | | CHG | 0.1 |
| E-25 | AM-5 | 100 | none | | none | |

| | | | | | | |
|---|---|---|---|---|---|---|
| [a] AM-1 was lyophilized to produce a solid material | | | | | | |

**Table 5: Example Antimicrobial Adhesive Test Results**

| ***S. aureus* Example** | **Zone of Inhibition** | | **Microbial Kill** | | | |
|---|---|---|---|---|---|---|
| | ***S. aureus*** | ***P. aeruginosa*** | ***S. aureus*** | | ***P. aeruginosa*** | |
| | | | **15 min** | **24 hour** | **15 min** | **24 hour** |
| E-1 | 7 | 7 | 0 | 3 | 0 | 1 |
| E-2 | 7 | 7 | NT[a] | NT | NT | NT |
| E-3 | 7 | 7 | NT | NT | NT | NT |
| E-4 | 0 | 7 | NT | NT | NT | NT |
| E-5 | 11 | 7 | NT | NT | NT | NT |
| E-6 | 7 | 7 | 0 | 3 | 0 | 5 |
| E-7 | 7 | 7 | NT | NT | NT | NT |
| E-8 | 7 | 7 | NT | NT | NT | NT |
| E-9 | 7 | 7 | 2 | 4 | 0 | 6 |
| E-10 | 0 | 0 | NT | NT | NT | NT |
| E-11 | 7 | 7 | NT | NT | NT | NT |
| E-12 | 7 | 0 | 0 | 1 | 0 | 2 |
| E-13 | 0 | 0 | NT | NT | NT | NT |
| E-14 | 7 | 7 | NT | NT | NT | NT |
| E-15 | 9 | 7 | NT | NT | NT | NT |
| E-16 | 12 | 7 | NT | NT | NT | NT |
| E-17 | 13 | 7 | 0 | 5 | 1 | 6 |
| E-18 | NT | NT | NT | 5 | 4 | 6 |

| | | | | | | |
|---|---|---|---|---|---|---|
| [a] NT = Not tested | | | | | | |

**Table 6: Antimicrobial Adhesion Stability (E-18)**

| | **Adhesion to Steel (g/cm)** | | | |
|---|---|---|---|---|
| | **Initial** | **23°C / 50%RH** | **120°C** | **90°C / 90%RH** |
| **Initial** | **279** | | | |
| **Day 14** | | **290** | **268** | **279** |
| **Day 21** | | **301** | **268** | **290** |

**Table 7: Antimicrobial Stability (E-18)**

| | **Microbial Kill (24 hours)** | | |
|---|---|---|---|
| | **Initial** | **120°C** | **90°C / 90%RH** |
| ***S. aureus*** | **5** | **5** | **5** |
| ***P. aeruginosa*** | **5** | **3** | **5** |

**Table 8: Example Antimicrobial Adhesive Test Results**

| **Sample** | **Peel Adhesion** | **Microbial Kill, 90 minutes (*S. aureus*)** | **Zone of Inhibition (*S. aureus*)** |
|---|---|---|---|
| **E-19** | **32.4** | **4** | **26.4** |
| **E-20** | **39.1** | **4** | **29.4** |
| **E-21** | **52.5** | **4** | **29.4** |
| **E-22** | **50.2** | **4** | **26.4** |
| **E-23** | **54.7** | **4** | **29.4** |
| **E-24** | **58.0** | **4** | **29.4** |
| **E-25** | **58.0** | **0.7** | **0** |

Various features and aspects of the present disclosure are set forth in the following claims.

## Claims

1. A pressure-sensitive adhesive (PSA) system comprising:
an antimicrobial polymer having a plurality of pendant groups comprising the formula (I): 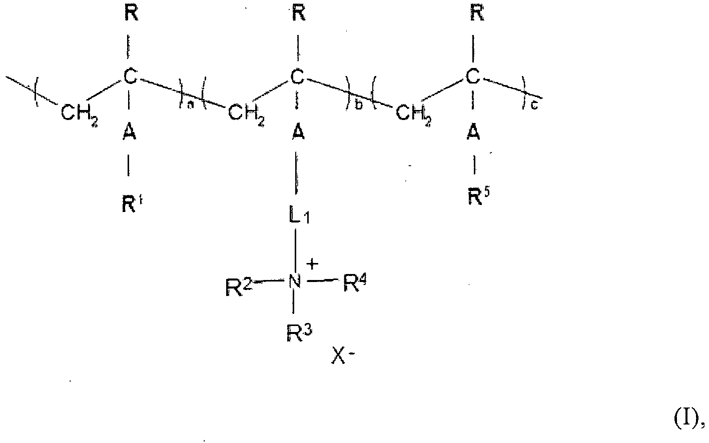 wherein:
each A is a carboxyl group, an ester group, an oxygen atom, a urethane group, a urea group, an alkyl group having from 1 to 12 carbons, or an aryl group;
R₁ is an alkyl group having from 1 to 22 carbons, or an aryl group;
L₁ is an alkylene group having from 1 to 3 carbons;
R₂, R₃, and R₄ are each selected from a hydrogen atom, an alkyl group having from 1 to 22 carbons, or an aryl group;
X is a chloride, a bromide, a tetrafluoroborate, an N-fluorobis(trifluoromethanesulfonyl)imide, a trifluoromethanesulfonate, a nonafluorobutanesulfonate, a methylsulfate, a gluconate, or an acetate;
R₅ is a hydrogen atom, N(R₆)₂, Si(OR₆)₃;
R₆ is an alkyl group having from 1 to 12 carbons; and
a + b + c is at least 50;
wherein the antimicrobial polymer is free of non-ionic hydrophilic moieties; and
wherein the PSA system has a glass transition temperature T_{g} ranging from 0 to 50 degrees C.

2. The PSA system of claim 1, wherein the PSA system has a glass transition temperature ranging from 10 to 40 degrees C.

3. The PSA system of claim 1 or 2, further comprising at least one of a tackifier and a plasticizer.

4. The PSA system of any of claims 1-3, further comprising an antimicrobial agent dispersed within the PSA system.

5. The PSA system of claim 4, wherein the antimicrobial agent includes at least one of chlorhexidine gluconate, chlorhexidine digluconate, polyhexylmethyl biguanide, a quaternary ammonium compound, silver, and a combination thereof.

6. The PSA system of claim 4 or 5, wherein the PSA system includes no greater than 5 wt% of the dispersed antimicrobial agent, based on the total weight of the PSA system.

7. The PSA system of any of claims 1-6, further comprising an adhesive polymer.

8. The PSA system of claim 7, wherein the PSA system includes a concentration of the adhesive polymer of at least 30 wt% based on the total weight of the PSA system.

9. The PSA system of any of claims 1-8, wherein the antimicrobial polymer comprises a polyacrylate.

10. The PSA system of any of claims 1-9, wherein the antimicrobial polymer is derived from a plurality of monomers, including monomers having the formula: 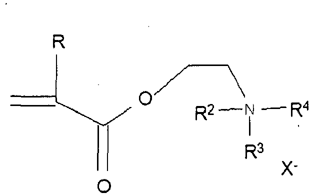 wherein:
R is selected from H and CH₃;
R₂, R₃, and R₄ are each selected from a hydrogen atom (-H), an alkyl group having from 1 to 22 carbons, or an aryl group; and
X is a chloride (Cl), a bromide (Br), a tetrafluoroborate (BF₄), an N-fluorobis(trifluoromethanesulfonyl)imide (N(SO₂CF₃)₂), a trifluoromethanesulfonate (OSO₂CF₃), a nonafluorobutanesulfonate (OSO₂C₄F₉), a methylsulfate (OSO₃CH₃), a gluconate (C₆H₁₁O₇), or an acetate (C₂H₃O₂).

11. The PSA system of any of claims 1-10, wherein a first pendant group has the formula (II): 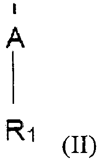 wherein A and R₁ are as defined in claim 1, and includes at least one of butyl, octyl, decyl, lauryl, bornyl, and octyldecyl.

12. A medical article comprising
a medical backing; and
the PSA system of any of claims 1-11 on a first surface of the backing.

13. The medical article of claim 12, further comprising a release liner in contact with the adhesive.

## Patentansprüche

1. Ein Haftklebemittel (PSA)-System, umfassend:
ein antimikrobielles Polymer, das eine Mehrzahl von anhängenden Gruppen aufweist, die die Formel (I) umfassen: 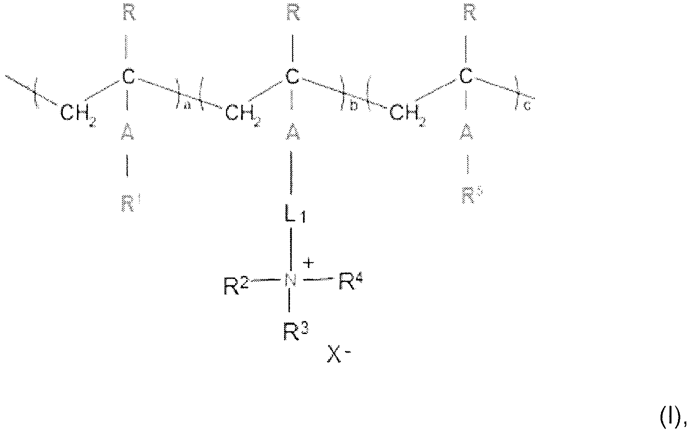 wobei:
jedes A für eine Carboxylgruppe, eine Estergruppe, ein Sauerstoffatom, eine Urethangruppe, eine Harnstoffgruppe, eine Alkylgruppe mit 1 bis 12 Kohlenstoffen oder eine Arylgruppe steht;
R₁ für eine Alkylgruppe mit 1 bis 22 Kohlenstoffen oder eine Arylgruppe steht;
L₁ für eine Alkylengruppe mit 1 bis 3 Kohlenstoffen steht;
R₂, R₃ und R₄ jeweils aus einem Wasserstoffatom, einer Alkylgruppe mit 1 bis 22 Kohlenstoffen oder einer Arylgruppe ausgewählt sind;
X für ein Chlorid, ein Bromid, ein Tetrafluorborat, ein N-Fluorbis-(trifluormethansulfonyl)imid, ein Trifluormethansulfonat, ein Nonafluorbutansulfonat, ein Methylsulfat, ein Gluconat oder ein Acetat steht;
R₅ für ein Wasserstoffatom, N(R₆)₂, Si(OR₆)₃ steht;
R₆ für eine Alkylgruppe mit 1 bis 12 Kohlenstoffen steht; und
a + b + c mindestens 50 beträgt;
wobei das antimikrobielle Polymer frei von nichtionischen hydrophilen Anteilen ist; und
wobei das PSA-System eine Glasübergangstemperatur T_{g} aufweist, die von 0 bis 50 °C reicht.

2. Das PSA-System nach Anspruch 1, wobei das PSA-System eine Glasübergangstemperatur aufweist, die von 10 bis 40 °C reicht.

3. Das PSA-System nach Anspruch 1 oder 2, ferner umfassend mindestens einen von einem Klebrigmacher und einem Weichmacher.

4. Das PSA-System nach einem der Ansprüche 1 bis 3, ferner umfassend ein antimikrobielles Mittel, das in das PSA-System dispergiert ist.

5. Das PSA-System nach Anspruch 4, wobei das antimikrobielle Mittel mindestens eines von Chlorhexidingluconat, Chlorhexidindigluconat, Polyhexylmethylbiguanid, einer quartären Ammoniumverbindung, Silber, und einer Kombination davon enthält.

6. Das PSA-System nach Anspruch 4 oder 5, wobei das PSA-System nicht mehr als 5 Gew.-% des dispergierten antimikrobiellen Mittels, bezogen auf das Gesamtgewicht des PSA-Systems, enthält.

7. Das PSA-System nach einem der Ansprüche 1 bis 6, ferner umfassend ein Klebemittelpolymer.

8. Das PSA-System nach Anspruch 7, wobei das PSA-System eine Konzentration des Klebemittelpolymers von mindestens 30 Gew.-%, bezogen auf das Gesamtgewicht des PSA-Systems, enthält.

9. Das PSA-System nach einem der Ansprüche 1 bis 8, wobei das antimikrobielle Polymer ein Polyacrylat umfasst.

10. Das PSA-System nach einem der Ansprüche 1 bis 9, wobei das antimikrobielle Polymer aus einer Mehrzahl von Monomeren abstammt, die Monomere mit der Formel enthalten: 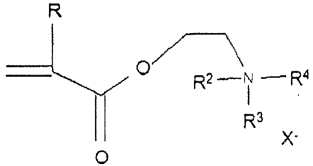 wobei:
R aus H und CH₃ ausgewählt ist;
R₂, R₃ und R₄ jeweils aus einem Wasserstoffatom (-H), einer Alkylgruppe mit 1 bis 22 Kohlenstoffen oder einer Arylgruppe ausgewählt sind; und
X für ein Chlorid (Cl), ein Bromid (Br), ein Tetrafluorborat (BF₄), ein N-Fluorbis-(trifluormethansulfonyl)imid (N(SO₂CF₃)₂), ein Trifluormethansulfonat (OSO₂CF₃), ein Nonafluorbutansulfonat (OSO₂C₄F₉), ein Methylsulfat (OSO₃CH₃), ein Gluconat (C₆H₁₁O₇) oder ein Acetat (C₂H₃O₂) steht.

11. Das PSA-System nach einem der Ansprüche 1 bis 10, wobei eine erste anhängende Gruppe die Formel (II) aufweist: 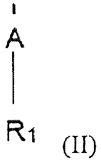 wobei A und R₁ wie in Anspruch 1 definiert sind, und mindestens eines von Butyl, Octyl, Decyl, Lauryl, Bornyl und Octyldecyl einschließen.

12. Ein medizinischer Gegenstand, umfassend
einen medizinischen Träger; und
das PSA-System nach einem der Ansprüche 1 bis 11 auf einer ersten Oberfläche des Trägers.

13. Der medizinische Gegenstand nach Anspruch 12, ferner umfassend eine Trennfolie, die mit dem Klebemittel in Kontakt steht.

## Revendications

1. Système d'adhésif sensible à la pression (ASP) comprenant :
un polymère antimicrobien ayant une pluralité de groupes pendants comprenant la formule (I) : 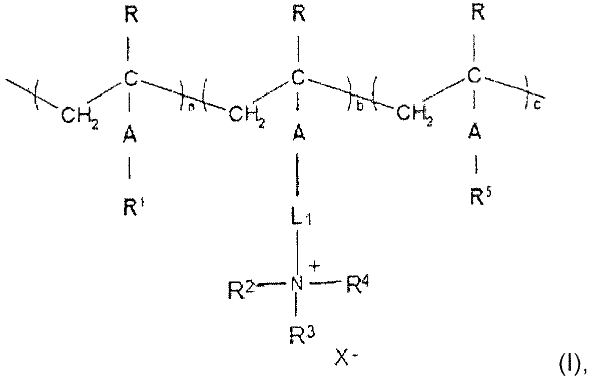 dans laquelle :
chaque A est un groupe carboxyle, un groupe ester, un atome d'oxygène, un groupe uréthane, un groupe urée, un groupe alkyle ayant de 1 à 12 carbones, ou un groupe aryle ;
R₁ est un groupe alkyle ayant de 1 à 22 carbones, ou un groupe aryle ;
L₁ est un groupe alkylène ayant de 1 à 3 carbones ;
R₂, R₃ et R₄ sont choisis chacun parmi un atome d'hydrogène, un groupe alkyle ayant de 1 à 22 carbones, ou un groupe aryle ;
X est un chlorure, un bromure, un tétrafluoroborate, un N-fluorobis(trifluorométhane-sulfonyle)imide, un trifluorométhanesulfonate, un nonafluorobutanesulfonate, un méthylsulfate, un gluconate, ou un acétate ;
R₅ est un atome d'hydrogène, N(R₆)₂, Si(OR₆)₃ ;
R₆ est un groupe alkyle ayant de 1 à 12 carbones ; et
a + b + c est d'au moins 50 ;
dans lequel le polymère antimicrobien est exempt de fragments hydrophiles non ioniques ; et
dans lequel le système d'ASP a une température de transition vitreuse T_{g} allant de 0 à 50°C.

2. Système d'ASP selon la revendication 1, dans lequel le système d'ASP a une température de transition vitreuse allant de 10 à 40°C.

3. Système d'ASP selon la revendication 1 ou 2, comprenant en outre au moins l'un d'un agent poisseux et d'un plastifiant.

4. Système d'ASP selon l'une quelconque des revendications 1 à 3, comprenant en outre un agent antimicrobien dispersé au sein du système d'ASP.

5. Système d'ASP selon la revendication 4, dans lequel l'agent antimicrobien inclut au moins l'un du gluconate de chlorhexidine, du digluconate de chlorhexidine, du polyhexylméthyle biguanide, d'un composé d'ammonium quaternaire, de l'argent et d'une combinaison de ceux-ci.

6. Système d'ASP selon la revendication 4 ou 5, dans lequel le système d'ASP n'inclut pas plus de 5 % en poids de l'agent antimicrobien dispersé, en fonction du poids total du système d'ASP.

7. Système d'ASP selon l'une quelconque des revendications 1 à 6, comprenant en outre un polymère adhésif.

8. Système d'ASP selon la revendication 7, dans lequel le système d'ASP inclut une concentration du polymère adhésif d'au moins 30 % en poids en fonction du poids total du système d'ASP.

9. Système d'ASP selon l'une quelconque des revendications 1 à 8, dans lequel le polymère antimicrobien comprend un polyacrylate.

10. Système d'ASP selon l'une quelconque des revendications 1 à 9, dans lequel le polymère antimicrobien est dérivé d'une pluralité de monomères, incluant des monomères ayant la formule : 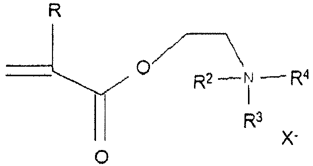 dans laquelle :
R est choisi parmi H et CH₃ ;
R₂, R₃ et R₄ sont choisis chacun parmi un atome d'hydrogène (-H), un groupe alkyle ayant de 1 à 22 carbones, ou un groupe aryle ; et
X est un chlorure (Cl), un bromure (Br), un tétrafluoroborate (BF₄), un N-fluorobis(trifluorométhanesulfonyle)imide (N(SO₂CF₃)₂), un trifluorométhanesulfonate (OSO₂CF₃), un nonafluorobutanesulfonate (OSO₂C₄F₉), un méthylsulfate (OSO₃CH₃), un gluconate (C₆H₁₁O₇), ou un acétate (O₂H₃O₂).

11. Système d'ASP selon l'une quelconque des revendications 1 à 10, dans lequel un premier groupe pendant a la formule (II) : 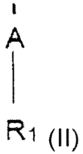 dans laquelle A et R₁ sont tels que définis dans la revendication 1, et incluen au moins l'un de butyle, octyle, décyle, lauryle, bornyle et octyldécyle.

12. Article médical comprenant
un support médical ; et
le système d'ASP selon l'une quelconque des revendications 1 à 11 sur une première surface du support.

13. Article médical selon la revendication 12, comprenant en outre une feuille anti-adhésive en contact avec l'adhésif.
